# EUROPEAN PATENT APPLICATION

(11) **EP 1 493 823 A1**
(43) Date of publication of application: **05.01.2005**
(21) Application number: 03077075.4
(22) Date of filing: 02.07.2003
(51) Int. Cl.: C12Q 1/68

(54) **HMG coenzyme A reductase inhibitors affect the mitochondrial DNA content of cells**

(71) Applicant: PrimaGen Holding B.V., 1105 BA Amsterdam (NL)
(72) Inventor: van Gemen, Bob, 1326 HV Almere (NL); de Baar, Marinus Petrus, 1011 TB Amsterdam (NL)
(74) Representative: Prins, Adrianus Willem, Mr. Ir.

(57) **Abstract**

Statins are widely used for the treatment of high cholesterol levels. In the present invention the effect of statins on mitochondrial nucleic acid and the activation state of mitochondria is used in methods for determining whether a subject is at risk of developing side effects of statin treatment, methods for the treatment of a clinical symptom associated with reduced mitochondrial function. Further provided are kits and the like comprising a means for the detection of mitochondrial nucleic acid, or the activation of a mitochondrion for use in a method mentioned above.

## Description

The invention relates to the field of medicine. More in particular, the invention relates to the field of diagnostics and pharmacology.

HMG coenzyme A reductase inhibitors, also referred to as statins, are presently widely used to treat high cholesterol levels. Statins are very effective in getting the cholesterol level into the desired range, and they have had thus far few side effects. With their increased use, and in particular with the recent clinical failure of cerivastatin, more of the side effects with prolonged use have become apparent.

In a minority of the patients some side effects are detected after prolonged treatment with statins. Side effects are diverse and differ from patient to patient. Most prevalent are hepato-toxicity and myopathy. The grade of side effect may also vary. The observed toxicity is typically dose dependent and reversible upon cessation of treatment. Several risk factors have been identified that are associated with a higher prevalence of side effects. These risk factors vary but include patients with another disease and patients undergoing severe trauma or heavy surgery.

Statins act through a common pathway. They all inhibit the activity of HMG coenzyme A reductase. This enzyme is crucial in an early step of the cholesterol biosynthesis pathway. By inhibiting this biosynthesis, most if not all the endogenous cholesterol syntheses capacity can be turned of, thereby contributing significantly to a general lower cholesterol level in the circulation. HMG coenzyme A reductase is a cytoplasmic enzyme that utilizes Acetyl-CoA as one of its substrates. Acetyl CoA can be generated from various sources but is typically made available to the enzyme via the mitochondrion of the cell.

In the present invention it was found that statins have a pronounced effect on the mitochondrial nucleic acid content of a cell. The cells of subjects that are treated with statins in dose ranges that are suitable for the treatment of high cholesterol levels. As a result of this treatment the mitochondrial nucleic acid content rises in the cell. Mitochondrial nucleic acid content is thus indicative for the use of statin by a subject. Thus in one aspect the invention provides a method for monitoring a subject receiving a HMG CoA reductase inhibitor said method comprising determining in a sample of said subject a first parameter indicative for the mitochondrial nucleic acid content of said sample. The parameter may be used as an absolute value or may be related to, for instance, the sample volume obtained or another parameter capable of placing the first parameter in a context. In preferred embodiment a second parameter indicative for the cellular content of the sample is determined. The two parameters can be related to one another to arrive at a parameter that reflects the relative mitochondrial nucleic acid content per cell in the sample. The mitochondrial nucleic acid content per cell ratio is a more accurate predictor of the action of statin in the body of the subject. The method may of course also be used in vitro, for instance in cell lines. Some statins require metabolic conversion prior to becoming active as a HMG coenzyme A reductase. This metabolic conversion is typically done by tissue cells of the subject, particularly the liver. Thus when the method is performed in vitro with statins requiring metabolic conversion it is preferred to use the converted statin directly or make metabolic conversion available in the in vitro system. A person skilled in the art is able to design suitable tests for his/her purpose. Thus while a method of the invention is preferably performed with at least two samples, where at least one is obtained prior to initiation of treatment, the person skilled in the art is capable of adjusting the experimental design to his/her special needs. It is for instance possible to compare the mitochondrial nucleic acid with historical controls. In a preferred embodiment a value obtained for at least a first parameter indicative for the mitochondrial nucleic acid content of said sample is compared with a reference. The reference can be a historical control or a sample obtained prior to initiation of treatment. There are however, many other particular designs possible which are also encompassed in the present invention.

The parameter for the mitochondrial nucleic acid content is preferably determined on the basis of mitochondrial DNA. Mitochondrial RNA can also be used. However, the parameter for the mitochondrial nucleic acid content may also be an indirect parameter such as for instance the mitochondrial volume or activity in the cell. Particularly the mitochondrial RNA or protein content is a parameter for the functionality and activity of the mitochondria and may thus also be used to moniter a subject receiving a HMG CoA reductase inhibitor. Similarly the parameter for the cellular content of the sample may be inferred through indirect parameters. However, preferably it is performed by means of determining the cellular nucleic acid content. The nucleic acid content of cell and/or mitochondrion can be determined in its entirety, thus for instance all mitochondrial nucleic acid may be determined or all nucleic acid of the cell compared with the mitochondrial nucleic acid content of the cell. As mentioned many different parameters may be determined and used to set of the mitochondrial nucleic acid content of sample. In a preferred embodiment the copy number of mitochondrial versus cellular DNA is determined. The copy number is an accurate measure of the mitochondrial nucleic acid of a cell.It is not necessary to determine this content in its entirety. One may measure just a part of the nucleic acid in the mitochondrion or cell. As the cellular genome varies between 1 (diploid) copy to two (diploid) copies per cell, one may also estimate the relative number of copies of the genome based on a value for only part of the genome, say (a part of) a gene. Similarly, the mitochondrial genome is present in a certain number of copies a cell. Thus not all mitochondrial DNA need be measured. It suffices to determine a parameter for this content based on only part of the mitochondrial genome, say a suitable gene. The same holds true when RNA is used to determine the parameter. A specific RNA or part thereof may be used as an indication for what occurs with the total RNA content of the cell or the mitochondrion. Many methods are suitable to measure mitochondrial nucleic acid versus cellular nucleic acid, or nuclear nucleic acid. For example suitable methods for determining the ratio of mitochondrial versus cellular nucleic acid are described in PCT/NL01/00883 and PCT/US01/147223, which are incorporated by reference herein.

The method may be used to determine whether a subject is undergoing statin treatment. However, the method can also be used to monitor a subject receiving statin treatment. An increase in the mitochondrial nucleic acid content of a cell is indicative for the number and/or the activation of mitochondria in the cell. The more mitochondrial nucleic acid, the more mitochondrions are in the cell and/or the the higher the activity is of the mitochondria in the cell. In the present invention it was found that cells normally respond to statins in the environment by increasing the number of mitochondria and/or activating their mitochondria. Subjects at risk of developing side effects from the treatment with statins, however, respond in an abnormal manner,. or are at least in part abnormal in responding in this manner. This abnormal behaviour is apparent prior to clinical manifestation of side effects. It is thus possible to determine if a subject is at risk of developing side effects from a statin treatment, by determining whether the mitochondrial nucleic acid content of a sample obtained from said subject displays the marked increase in response to the treatment within a normal range observed in individuals with effective statin treatment without side effects. If the increase is absent or not sufficiently high, the subject is at risk. Alternatively, the increase may be abnormally high, indicative for an abnormal high mitochondrial activity and/or volume, which can result in overheating of the mitochondria and subsequent loss of mitochondrial functional and eventually mitochondrial death and lysis.

The method can be performed at any stage prior, during or after treatment of the subject with a statin. If done before, the method is preferably performed in vitro, as mentioned above. Thus the invention further provides a method of the invention further comprising determining from said first and/or second parameter whether said subject is developing a side effect from said inhibitor, or is at risk of developing such a side effect. In cases where it is determined that the subject is at risk, the treatment of the subject may be altered as a result of said risk. The alteration may comprise additional treatment to at least in part prevent a side effect of the statin treatment. However, preferably, the alteration comprises the cessation of the statin treatment.

The sample may be obtained from any part of the body of the subject. As mentioned above, statins are very hepatotrophic. On the other hand, a major side effect of statin treatment occurs in the muscle tissue of the subject. The sample may thus comprise a liver or muscle sample. Samples may also be taken from other parts of the subject. Preferably, the sample comprises blood cells. The sample is preferably a cellular sample that can be obtained in a non-invasive manner. Blood samples are typically regarded as non-invasive samples and are for the present invention preferred. Non-invasive cellular samples can also be obtained from other parts of the body of a subject and include but are not limited to mucosal surface samples such as from the mouth. The mitochondrial nucleic acid content and/or cellular content are preferably determined in the peripheral blood mononuclear cell (PBMC) fraction of the blood sample. Such samples are routinely generated in a clinical lab.

A change of the mitochondrial nucleic acid content of a cell is, as mentioned above, indicative for a change in the activation state of the mitochondria in the cell. Thus by providing a cell with a statin it is possible to enhance mitochondrial function in the cell. This embodiment of the invention is useful for the treatment of subjects suffering from diseases related to, or associated with a reduced mitochondrial function. Thus the invention provides a method for at least in part reducing a clinical symptom related with a reduced mitochondrial function in a (part of a) subject, said method comprising administering a HMG CoA reductase inhibitor to said subject. Such clinical symptoms preferably comprise fatigue, (peripheral) neuropathy, (cardio) myopathy, lactic acidosis, liver failure, lipodistrophy, lipoatrophy, heart disease, hepatic steatosis, diabetes, Osteoarthritis, infertility problems, Parkinson's disease or viral infection like HIV-1, HCV, HBV. It is also possible to treat subjects that are at risk of developing a symptom associated with or related to a reduced mitochondrial function. Statins are preferably used for the treatment of subjects undergoing treatment with a nucleotide analogue. Such analogues are typically used in the treatment of virus infection. The treatment is based on the inhibition of virus-replication through interference with nucleic acid polymerases specific for the virus. Such analogues have as a common side effect that they also inhibit cellular polymerases including those polymerases specific for cellular organelles containing their own nucleic acid, such as mitochondria and chloroplasts. This inhibition cellular polymerases is one of the causes of side effects in the treatment with nucleotide analogues. The present invention provides a means to boost at least mitochondrial nucleic acid content of a cell and thereby counteracts the action of the nucleotide analogue at least to some extend. Preferred nucleotide analogues are .... The invention thus further provides a metod for the treatment of a subject receiving a nucleotide analogue comprising providing said subject with a statin.
The statin is be given in dosages effective to raise the mitochondrial nucleic acid content in a sample taken from said subject. Effective dosages vary from statin to statin and generally overlap the range effective in the reduction of cellular cholesterol synthesis in a subject. Statins that may be used for the present invention comprise Pravastatin (Pravachol™), Lovastatin (Mevacor™), Simvastatin (Zocor™), Fluvastatin (Lescol™), Atorvastatin (Lipitor™), Cerivastatin (Baycol™), Rosuvastatin (Crestor™) or Lovastatin (Advicor™). The above mentioned statins are preferred because they are already on the market (with the exception of Cerivastatin). However, it may be clear that any compound with HMG coenzyme A reductase inhibiting activity may be used in the present invention. Such compounds may be a functional part, derivative and/or analogue of a preferred statin as mentioned above. However, it may also be a different compound with the same HMG coenzyme A reductase activity in kind, not necessarily in amount. In a preferred embodiment the statin comprises Simvastatin or a functional part, derivative and/or analogue thereof.

### Examples

### Example 1

Experiment: seven patients between 43 and 62 years old and all with elevated total cholesterol levels started using 20 mg simvastatin per day for a period of 2 months to reduce their cholesterol levels. Three control patients with the same indication used fibric acid derivatives to reduce their cholesterol levels. In total the study included x male and y female patients. At day 0 and after 2 months, whole blood samples were taken using CTP tubes (Beckton-Dickinson) and PBMC were purified out of these samples using standard protocols provided by the manufacturer of the tubes. The samples were checked for the necessary absence or low levels of trombocytes (less than 5 per cell). All samples fulfilled these criteria. In these samples the mtDNA content per PBMC was determined using the Primagen Retina MiTox DNA assay.

Nucleic acids were isolated from 10⁵ PBMC according to the method described by Boom et al. and dissolved in 50 µl DNAse and RNAse free water. Five µl of the nucleic acid (equivalent to 1,000 PBMC) was put in the reaction mix to amplify the specific targets. In parallel, 10³ molecules of plasmid containing Snrp DNA was mixed with 4 x 10⁵, 2 x 10⁵, 10⁵, or 5 x 10⁴ molecules of plasmid containing mitochondrial DNA, and the mixture was used as input for the reactions. Reactions were performed in a 20µl reaction volume and contained: 40mM Tris-pH 8.5, 90mM KCl, 12mM MgCl2, 5mM dithiotreitol, 1mM dNTP's (each), 2mM rNTP's (each), 0.2µM primer (each), 0.05µM molecular beacon, 1.5 units restriction enzyme Msp I, 375mM sorbitol, 0.105 µg/µl bovine serum albumin, 9.6 units AMV RT, 64 units T7 RNA polymerase, 0.08 units RNAse H and input nucleic acid. The complete mixture, except the enzymes, sorbitol and bovine serum albumin was, prior to adding the enzyme mixture, incubated at 37°C for 25 minutes and subsequently heated to 95°C for two minutes in order to denature the DNA and to allow the primers to anneal. After cooling the mixture to 41°C the enzyme mixture was added. The amplification took place at 41°C for 90 min in a fluorimeter (Primagen Retina Reader) and the fluorescent signal was measured every minute (using the filter set 530/25 nm and 485/30 nm).
The reaction mix (duplex-mix) contained two sets of primers and beacon:
SnrpD p1 and SnrpD p2 (for nuclear DNA, each 0.2 µM), and MtD p1 and MtD p2 (for mitochondrial DNA, each 0.2 µM) with beacons SnrpD mb (ROX-labeled) and MtD mb (FAM-labeled) (each 0.04 µM).
In a duplex reaction with two competing amplifications the ratio of the slope of the curves of fluorescence in time is proportional to the ratio of the amount of molecules of each amplified species. The data of the plasmid Snrp / mitochondrial DNA mixtures were used to create a standard curve on which the unknown ratio of mitochondrial to Snrp nuclear DNA of the PBMC samples could be assessed.

### Name Sequence 1

MtD p1 5' AAT TCT AAT ACG ACT CAC TAT AGG GAA GAA CCG GGC
TCT GCC ATC TTA A 3'

MtD p2 5' GTA ATC CAG GTC GGT TTC TA 3'

MtD mb 5' CGT ACG TGA TAT CAT CTC AAC TTA GTA TCG TAC G

SnrpD p1 5'
AATTCTAATACGACTCACTATAGGGAGAGGCCCGGCATGTGGTGCATAA 3'

SnrpD p2 5' TGC GCC TCT TTC TGG GTG TT 3'

SnrpD mb 5' CGC ATG CTG TAA CCA CGC ACT CTC CTC GCA TGC G 3'

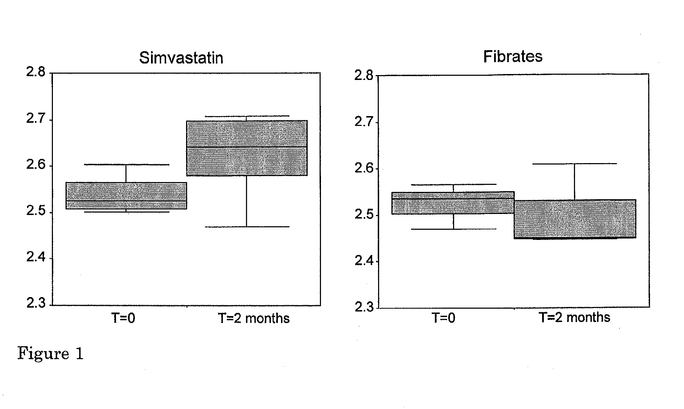

The results (see figure 1) were analysed using the paired samples T-test as implemented in SPSS for Windows, version 11.5.1 (SPSS Inc., Chicago, IL, U.S.A.). Normal distribution of mtDNA data is obtained after a 10th base logarithm transformation of mtDNA copies per cell. The p-value resulting from the paired samples T-test on the logarithmically transformed data was 0.045 with higher mtDNA values after 2 months relative to baseline values. This indicates a significantly increasing mtDNA content due to statin use. No changes were observed in the control group of 3 patients treated with fibric acid derivatives (fibrate), (p=0.737). In this latter group there could be more a trend towards decrease of mtDNA content, but to state this, larger groups should be treated and analysed.
We also observed a trend towards increase in mtRNA due to the use of statines, but this trend was not as strong as observed for mtDNA.
Simvastatin is a potent inhibitor of HMG-CoA reductase, thereby inhibiting the synthesis of cholesterol, but also intermediate products and products that use cholesterol as base, or share parts of the biosynthesis pathway like Co-enzyme Q10. Co-enzyme Q10 is involved in the oxidative respiratory chain in the mitochondrion. Since the mitochondria are involved in the synthesis of part of the proteins involved in the oxidative respiratory chain, increased levels of mtDNA as observed in the studied individuals suggest that the cell is compensating its loss in oxidative capacity and probably also other functions by increased synthesis of both mtDNA and so probably its functional proteins.

### LEGENDS TO FIGURES

Figure 1. Median and interquartile mitochondrial DNA values in PBMC of patients treated with simvastatin (left panel) or fibrates (right panel). Compared were values measured at the start of the therapy (T=0) and after two months on the respective therapy (T=2 months). Clearly simvastatin causes an increase in the number of mitochondrial DNA copies in PBMC compared to fibrates.

## Claims

1. A method for monitoring a subject receiving a HMG CoA reductase inhibitor said method comprising determining in a sample of said subject a first parameter indicative for the mitochondrial nucleic acid content of said sample.

2. A method according to claim 1, further comprising determining in said sample a second parameter indicative for the cellular content.

3. A method according to claim 1 or claim 2, wherein at least one of said parameters is compared with a reference.

4. A method according to any one of claims 1-3, further comprising determining from said first and/or second parameter whether said subject is developing a side effect from said inhibitor, or is at risk of developing such a side effect.

5. A method according to any one of claims 1-4, further comprising altering the treatment of said subject.

6. A method for at least in part reducing a clinical symptom related with a reduced mitochondrial function in a (part of a) subject, said method comprising administering a HMG CoA reductase inhibitor to said subject.

7. A method according to claim 6, wherein said subject is suffering from or at risk of suffering from fatigue, (peripheral) neuropathy, (cardio) myopathy, lactic acidosis, liver failure, lipodistrophy, lipoatrophy, heart disease, hepatic steatosis, diabetes, Osteoarthritis, infertility problems, Parkinson's disease or a viral infection.

8. A method according to claim 7, wherein said viral infection is HIV-1, HCV or HBV.

9. A method according to any of claims 6-8, wherein said subject is under treatment with a nucleotide analogue.

10. Use of a HMG CoA reductase inhibitor for the preparation of a medicament for the treatment of mitochondrial dysfunction in a subject.

11. Use of a HMG CoA reductase inhibitor for at least in part improving mitochondrial function in a cell cultured.

12. Use according to claim 11, wherein said cell is cultured *in vitro.*

13. A method for determining whether a subject is at risk of suffering from a side effect of a treatment with a HMG CoA reductase inhibitor comprising determining in a cellular sample from said subject whether mitochondrial nucleic acid is affected in response to *in vitro* exposure to said HMG reductase inhibitor.

14. Use of a kit for determining a mitochondrial nucleic acid in a sample for monitoring a subject receiving a HMG CoA reductase inhibitor.
